# FASCICULE DE BREVET EUROPEEN

(11) **EP 1 008 576 B1**
(45) Date de publication et mention de la délivrance du brevet: **10.12.2003**
(21) Numéro de dépôt: 99402479.2
(22) Date de dépôt: 08.10.1999
(51) Int. Cl.: C07C 17/38, C07C 17/389, C07C 19/08, B01J 20/34

(54) **Procédé de séchage du difluorométhane**
Verfahren zur Trocknung von Difluormethan
Method for drying difluoromethane

(30) Priorité: 08.12.1998 FR 9815469
(43) Date de publication de la demande: 14.06.2000
(73) Titulaire: Atofina, 92800 Puteaux (FR)
(72) Inventeur: Bertocchio, René, 69390 Vourles Par Vernaison (FR)

(56) Documents cités:
- WO-A-91/15445
- FR-A- 2 657 870
- DATABASE WPI Section Ch, Week 8434 Derwent Publications Ltd., London, GB; Class E16, AN 84-207506 XP002110554 & DD 209 182 A (VEB CHEMIEKOMB BITT), 25 avril 1984 (1984-04-25)
- DATABASE WPI Section Ch, Week 9637 Derwent Publications Ltd., London, GB; Class E16, AN 96-366140 XP002110555 & JP 08 173799 A (TOSOH CORP), 9 juillet 1996 (1996-07-09)

## Description

La présente invention concerne le domaine des hydrocarbures fluorés et a plus particulièrement pour objet un procédé de séchage en continu de difluorométhane (CH₂F₂) humide, mettant en oeuvre un tamis moléculaire, de type A, et utilisable dans une unité de production industrielle.

Le difluorométhane (connu dans le métier sous la désignation F32 ou HFC-32) est l'un des substituts possibles aux chlorofluorocarbures (CFC) concernés par le Protocole de Montréal. Il est destiné à remplacer plus particulièrement le chloropentafluoroéthane (F115, dont l'action sur l'ozone s'accompagne d'une contribution très forte à l'effet de serre) et, dans un proche avenir, le F22 ou chlorodifluorométhane. Il entre à ce titre dans la composition de plusieurs mélanges à caractère quasi-azéotropique, tels que le R407 C (mélange avec le HFC-125 ou pentafluoroéthane à raison de 50 % / 50 % en poids) ou le R410 A (mélange HFC-32/HFC-125 ou pentafluoroéthane/HFC-134a ou 1,1,1,2-tétrafluoroéthane à raison de 23 % / 25 % / 52 % en poids), utilisés dans l'industrie du froid.

Le F32 peut être obtenu par fluoration du chlorure de méthylène (CH₂Cl₂) au moyen de fluorure d'hydrogène (HF) en présence d'un catalyseur ou par hydrogénolyse du dichlorodifluorométhane (F12) ou du chlorodifluorométhane (F22) ou encore par décomposition, en présence d'HF, des éthers alphafluorés sous l'action d'acides de Lewis.

Certains de ces procédés nécessitent des lavages acides ou basiques qui introduisent des quantités plus ou moins importantes d'eau dans le produit final. Ce dernier doit donc subir une opération supplémentaire de séchage pour satisfaire les spécifications normalement fixées pour les hydrofluorocarbures (HFC), c'est-à-dire moins de 10 ppm d'humidité. Une telle spécification est nécessaire pour éviter les problèmes de corrosion dans les machines frigorifiques.

Les tamis moléculaires, encore appelés zéolithes synthétiques, sont des composés chimiques largement utilisés dans l'industrie comme agents adsorbants, notamment pour sécher des gaz ou des liquides. Ce sont des alumino-silicates métalliques qui possèdent une structure cristalline tridimensionelle constituée par un assemblage de tétraèdres. Ces tétraèdres sont formés par quatre atomes d'oxygène qui occupent les sommets, et qui entourent soit un atome de silicium, soit un atome d'aluminium placé au centre. Ces édifices contiennent généralement des cations pour rendre le système électriquement neutre, tels ceux dérivés du sodium, du potassium ou du calcium.

Dans le cas des tamis moléculaires, dits du type A, les tétraèdres sont assemblés de telle manière qu'ils composent un octaèdre tronqué. Ces octaédres sont eux mêmes arrangés selon une structure cristalline cubique simple, formant un réseau dont les cavités ont un diamètre approximatif de 11,5 Å. Ces cavités sont accessibles par des ouvertures, ou pores, qui peuvent être partiellement bloquées au moyen de cations. Lorsque ces cations sont dérivés du sodium, ces cavités ont un diamètre d'ouverture de 4,1 Å, et l'on a alors un tamis moléculaire dit 4 A. La structure cristalline d'un tel tamis peut être représentée par la formule chimique suivante :

Na₁₂[(AlO₂)₁₂(SiO₂)₁₂].X H₂O

dans lequelle X qui représente le nombre de molécules d'eau appartenant à la structure (eau de cristallisation) peut atteindre 27, ce qui représente 28,5 % en poids de la zéolithe anhydre.

Après élimination de l'eau de cristallisation par chauffage à une température de l'ordre de 500 à 700 °C, les cavités de ces substances sont disponibles pour l'adsorption sélective de différents gaz ou liquides. Ainsi, les pores des divers types de zéolithe ne permettent de laisser passer et de s'adsorber dans les cavités correspondantes que les molécules dont le diamètre effectif est inférieur ou égal au diamètre effectif des pores. Dans le cas du séchage de gaz ou de liquides, ce sont donc les molécules d'eau qui sont retenues par adsorption sélective à l'intérieur des cavités mentionnées précédemment, la substance à sécher n'étant quant à elle pas ou peu adsorbée.

La taille des ouvertures (ou des pores) peut du reste être modifiée selon les différents types de tamis moléculaire. Ainsi, par échange d'une grande partie des ions sodium d'un tamis moléculaire 4A par des ions potassium, on obtient le tamis moléculaire 3A, dont les pores ont un diamètre d'environ 3 Å. Le tamis moléculaire 5A est réalisé en remplaçant les ions sodium par des ions calcium, le diamètre effectif des pores étant alors de l'ordre de 5 Å.

Les tamis de type 3A, 4A ou 5A sont largement disponibles commercialement.

Sur un plan pratique, les tamis moléculaires peuvent être combinés avec d'autres substances telles que des liants, notamment des argiles, et les compositions obtenues sont façonnées par exemple en granulés, billes ou extrudés.

Les tamis moléculaires ainsi conditionnés, sont mis en oeuvre industriellement par chargement dans des colonnes séchantes, dans lesquelles est introduit le gaz humide, et d'où il ressort séché.

Au bout d'une certaine durée de fonctionnement dans une colonne séchante, qui varie avec les conditions opératoires (débit de gaz à sécher, quantité de tamis moléculaire), on observe une augmentation de la teneur en eau du gaz séché, en sortie de colonne. Ce moment correspond à l'atteinte de la capacité de saturation en eau de la charge de tamis, c'est-à-dire de la quantité maximale d'eau pouvant être adsorbée. Cette quantité se situe généralement à environ 20% en poids, exprimée par rapport au poids de tamis sec.

La charge de tamis ainsi saturée d'eau doit alors être soumise à un traitement dit de régénération, à l'issue duquel la capacité initiale du tamis à adsorber de l'eau est restaurée. Ce traitement consiste habituellement à faire passer dans la colonne un courant d'un gaz inerte, à une température comprise entre 200°C et 300 °C. Sur un plan pratique, ce traitement de la charge de tamis saturée s'effectue dans la même colonne que celle dans laquelle a été introduit le courant de gaz à sécher. Une même colonne séchante fonctionne donc tantôt en phase de séchage du gaz humide, tantôt en phase de régénération de la charge de tamis moléculaire par le gaz inerte. A l'issue d'un certain nombre de ces cycles séchage-régénération, on observe cependant une baisse irréversible de la capacité de saturation en eau de la charge de tamis, et il est alors nécessaire d'arrêter le fonctionnement de la colonne pour renouveler la charge de tamis par une charge fraîche.

On entend par charge fraîche de tamis, dans le présent texte, une charge de tamis qui n'a pas été utilisée comme agent séchant.

Dans les conditions de la pratique industrielle de séchage des gaz au moyen de tamis moléculaires, on utilise habituellement 2 colonnes de séchage qui peuvent fonctionner en alternance, l'une étant en phase de séchage tandis que l'autre est en phase de régénération.

Le séchage de F32 par tamis moléculaire pose un problème spécifique en raison de la proximité de diamètre effectif entre les molécules de F32 et d'eau (respectivement 0,33 nm et 0,21 nm).

Ainsi, la demande FR 2705586 mentionne bien la mise en contact dans un récipient sous pression de F32 humide avec un tamis moléculaire de type 3 A et une huile d'ester à une température de 120°C.

Ce document enseigne cependant que, dans ces conditions, le F32 s'adsorbe sur ledit tamis et subit une réaction de décomposition, laquelle a pour effet, via une modification de l'état cristallin du tamis, de diminuer fortement sa capacité de saturation en eau.

Ce document en conclut qu'un tel tamis n'est pas adapté à une utilisation comme agent séchant du F32. La demande de brevet recommande en conséquence, dans le but de sécher le F32 circulant comme agent réfrigérant à l'intérieur d'une machine frigorifique, un tamis moléculaire obtenu par un traitement complémentaire d'un tamis de type 3 A qui entraîne une diminution de la taille des pores.

Il a à présent été trouvé que cet inconvénient pouvait être évité en procédant à un séchage d'un courant de F32 produit en continu, dans un domaine particulier de température, et en mettant en oeuvre, notamment, un procédé spécifique de régénération de la charge de tamis.

Un but de la présente invention est donc de proposer un procédé de séchage de F32 humide, mettant en oeuvre un tamis moléculaire simple, disponible commercialement, et utilisable dans une unité de production industrielle de F32.

Un autre but de l'invention est de proposer un procédé de séchage en continu de F32 humide qui conduise à séparer sélectivement l'eau du F32, avec des pertes réduites en F32.

Un autre but de l'invention est de proposer un procédé de séchage en continu du F32 humide comprenant une étape de régénération de la charge de tamis moléculaire qui maintienne sensiblement constante sa capacité de saturation en eau.

Un autre but de l'invention est de proposer un procédé de séchage en continu du F32 humide permettant une réduction du temps durant lequel les colonnes de séchage sont arrêtées pour renouvellement de la charge de tamis moléculaire.

Il a à présent été trouvé que les buts pré-cités sont atteints, en totalité ou en partie, au moyen du procédé selon l'invention qui est décrit ci-après.

La présente invention concerne donc un procédé de séchage de F32 humide comprenant la mise en contact en continu d'un courant dudit F32 avec une charge d'une composition comprenant un tamis moléculaire choisi parmi un tamis de type 3 A, 4 A ou 5 A, à une température comprise entre 5 et 78°C, de préférence à température ambiante, et à une pression comprise entre 0,6 et 25 atm, de préférence entre 0,8 et 17 atm.

Contrairement à l'enseignement de l'art antérieur, il est donc possible, conformément à l'invention, d'utiliser des tamis de type A, qui sont disponibles commercialement, pour sécher du F32 en continu.

Le courant de F32 à sécher peut être un courant gazeux ou liquide. Lorsque le courant de F32 à sécher est liquide, on opère avantageusement à une pression comprise entre 9 et 25 atm, de préférence entre 12 et 17 atm.

Lorsque, selon une variante préférée, le courant de F32 à sécher est un gaz, on opère à une pression comprise entre 0,6 et 10 atm, de préférence entre 0,8 et 5 atm.

Le courant de F32 à sécher comprend généralement une teneur en eau inférieure à 10000 ppm, de préférence inférieure à 6000 ppm.

La mise en contact du F32 humide et de la charge de tamis est de préférence réalisée dans une colonne de séchage située dans la partie aval d'une unité de fabrication de F32.

Avant sa mise en oeuvre pour le séchage du courant de F32, la charge fraîche de tamis moléculaire est soumise à un traitement d'activation. Ce traitement a pour but d'éliminer l'humidité adsorbée après la fabrication du matériau durant son stockage et les manipulations précédant sa mise en place dans la colonne de séchage. Ce traitement comprend généralement un chauffage à une température comprise entre 200 et 300°C et à une pression voisine de la pression atmosphérique.

Le débit du courant de F32 à sécher et la quantité de charge de tamis adaptée au séchage peuvent être déterminés sans difficultés excessives par un homme du métier compétent en génie chimique par calcul et essais, en fonction de la taille de l'unité industrielle.

Selon une variante préférée du procédé selon l'invention, le tamis moléculaire mis en oeuvre est un tamis de type 3 A. Un tel tamis présente avantageusement, du fait de son diamètre effectif de pores, une capacité réduite d'adsorption de F32 et une efficacité améliorée.

Selon une variante préférée du procédé selon l'invention, la charge de tamis moléculaire mise en oeuvre est avantageusement régénérée (après avoir atteint sa capacité de saturation en eau), par le procédé consistant à chauffer ladite charge à une température comprise entre 120°C et 300°C, de préférence entre 150° et 250°C, à une pression absolue inférieure à 100 mm Hg, de préférence inférieure à 80 mm Hg. La durée de ce procédé est avantageusement déterminée de manière à désorber la quasi-totalité de la quantité de produits (essentiellement l'eau et, de façon minoritaire, le F32 résiduel) adsorbés à l'issue du séchage du F32 humide. On désigne cette quantité par l'expression "quantité initiale" dans les lignes qui suivent.

Selon une autre variante préférée du procédé selon l'invention, la charge de tamis moléculaire mise en oeuvre est régénérée par le procédé consistant à faire passer un courant d'un gaz inerte, tel que l'hélium, sur ladite charge, à une pression voisine de la pression atmosphérique, en opérant tout d'abord :
(i) à au moins une température comprise entre 70°C et 170°C, de préférence entre 80°C et 165 °C, durant le temps nécessaire à l'élimination d'au moins 80 %, de préférence au moins 90 %, de la quantité de F32 initiale adsorbée dans la charge, puis,
(ii) à une autre température comprise entre 180°C et 300°C, de préférence entre 190°C et 250°C, durant le temps nécessaire à l'élimination d'au moins 90 %, de préférence au moins 95 %, de la quantité d'eau initiale adsorbée dans la charge.

Le temps nécessaire de fonctionnement à la température (i) est déterminé par le suivi de l'évolution de la teneur en F32 du gaz inerte, à la sortie de la colonne de régénération, par des méthodes de contrôle appropriées, telle que par dosage chromatographique. Le temps nécessaire de fonctionnement à la température (ii) est déterminé de manière analogue, par exemple à l'aide d'un doseur d'humidité. Ces temps sont fonction d'un certain nombre de paramètres dépendant de l'installation et bien connus de l'homme du métier : débit du gaz inerte de balayage, chaleurs de désorption de l'eau et du F32, masse calorifique du tamis et de l'appareillage métallique renfermant le tamis.

Ces 2 dernières variantes de mises en oeuvre du procédé selon l'invention, portant sur les modalités de la régénération de la charge de tamis, sont particulièrement avantageuses. En effet, elles permettent, à l'issue de la régénération de maintenir la capacité de saturation en eau de la charge de tamis moléculaire à une valeur sensiblement égale à celle avant régénération. Ainsi, une même charge de tamis mise en oeuvre industriellement peut être utilisée de manière efficace dans un plus grand nombre de cycles : séchage de F32/régénération. Parmi ces deux variantes, celle mettant en oeuvre un courant de gaz inerte est plus particulièrement préférée pour sa réalisation et sa conduite plus simples dans une unité industrielle.

Lorsque l'on procède au traitement de régénération de la charge de tamis moléculaire par le procédé en 2 étapes qui vient d'être décrit, il est particulièrement avantageux de mettre en oeuvre l'étape (i) en opérant tout d'abord :
- (i1) à une première température comprise entre 70°C et 130°C, de préférence entre 100°C et 125°C, durant le temps nécessaire à l'élimination d'au moins 60 % (de préférence au moins 70 %) de la quantité de F32 initiale adsorbée, puis
- (i2) à une deuxième température comprise entre 130°C et 170°C, de préférence entre 145°C et 165°C, durant le temps nécessaire à l'élimination d'au moins 80 %, de préférence au moins 90 %, de la quantité de F32 initiale adsorbée.

Un tel traitement permet de maintenir encore mieux la capacité de saturation en eau de la charge de tamis. Il permet encore de récupérer du F32 dont la teneur en eau est considérablement abaissée par rapport à celle du F32 humide à sécher, notamment à l'issue de l'étape (i1).

Le traitement de régénération de la charge de tamis, conforme à l'une des deux variantes décrites précédemment, est avantageusement mis en oeuvre dans la même colonne que celle mentionnée ci-dessus. De manière encore plus avantageuse, le procédé de séchage selon l'invention est mis en oeuvre dans deux colonnes en parallèle, l'une fonctionnant en phase de séchage de F32 humide proprement dit, l'autre fonctionnant en phase de régénération d'une charge de tamis moléculaire saturée.

Dans le cas où, comme rappelé précédemment, on procède pour la régénération de la charge de tamis à un chauffage à une température comprise entre 200 et 300°C (en présence d'un courant de gaz inerte), on observe une dégradation de la capacité de saturation en eau de la charge de tamis moléculaire. Une telle dégradation conduirait à arrêter l'installation industrielle, pour renouveler la charge de tamis, dans des conditions incompatibles avec la conduite d'une installation industrielle de séchage.

Outre le tamis moléculaire, la composition mise en oeuvre dans le procédé selon l'invention comprend des additifs utilisés généralement dans ce domaine, notamment un agent de liaison argileux qui permet aux produits façonnés en zéolithe de conserver leur aptitude à être mis en forme et leur résistance. La composition se présente généralement sous forme de perles, ou de granulés. Du point de vue de leur résistance et de leur pouvoir de dessiccation effectif, il est souhaitable que les granulés, de forme essentiellement cylindrique, aient un diamètre de 0,5 à 5 mm et une longueur de 3 à 15 mm, et que les perles aient un diamètre de 1 à 5 mm.

Les exemples suivants sont donnés à titre purement illustratif, et non limitatif, du procédé selon l'invention.

### Exemple 1 :

### Séchage d'un courant de F32 par une charge de tamis moléculaire de type 3A

Une charge de 40,8 g de tamis Ceca NK 30 (3 Å), sous forme de granulés de diamètre voisin de 1,5mm et de longueur comprise entre 5 et 10 mm, est placée à l'intérieur d'un tube sécheur en acier inoxydable (6), d'un diamètre interne de 14 mm et de 750 mm de hauteur. Le sécheur présente ainsi une hauteur utile d'environ 380 mm et il est équipé d'une double enveloppe permettant le chauffage de la charge de tamis.

On procède au traitement préalable d'activation de la charge par chauffage à 200°C.

On fait circuler ensuite à travers ce tube sécheur au débit de 44 l/h, à une température d'environ 20°C et sous une pression de 1 atm, un courant gazeux de F32 renfermant 4100 ppm d'eau, et on suit l'efficacité du séchage par mesure de la teneur en eau réalisée à l'aide d'une cellule à conductivité électrique (7) couplée à un conductimètre (8) lui-même relié à un enregistreur (9) et à un dispositif d'arrêt automatique (10).

Le gaz sec traverse ensuite un réservoir tampon d'une capacité de 5 litres (15) d'où il est envoyé à l'aide d'une pompe à membrane (1) sur un humidificateur composé d'une colonne de billes de verre (3) et d'un pousse-seringue (4) qui introduit de l'eau liquide dans le système au débit de 0,4 ml/h, de telle sorte que le courant de F32 séché est de nouveau humidifié à la valeur de 4100 ppm d'eau, précitée. Après cette humidification et passage dans un volume d'homogénéisation (5), le courant retourne au sécheur (6).

Un volume tampon d'une capacité de 10 litres (11) permet de maintenir la pression du courant gazeux de F32 à une valeur voisine de 1 atm.

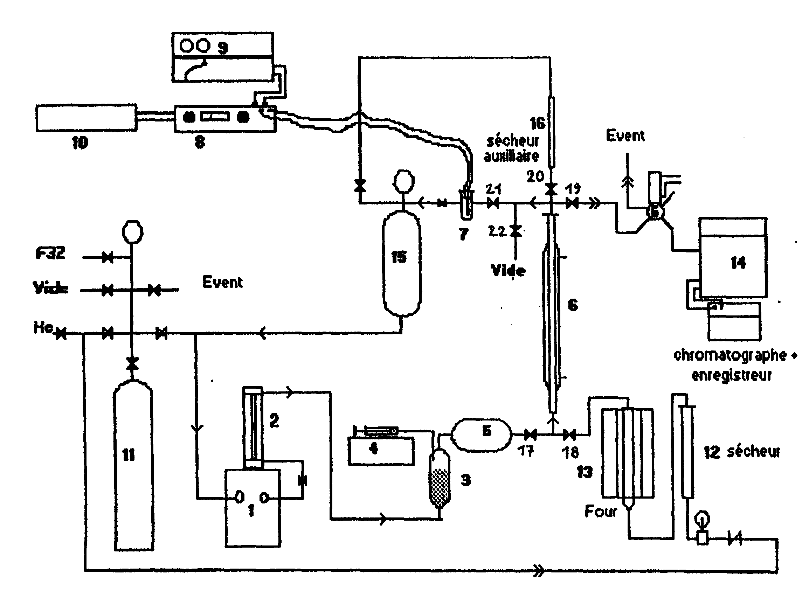

L'ensemble décrit sur la figure 1 constitue donc une boucle de séchage en phase gaz qui simule le fonctionnement d'une colonne de séchage traitant en continu un courant gazeux humide de F32.

En sortie du tube sécheur on mesure une teneur en eau du F32 inférieure à 10 ppm.

La déviation du signal de sortie de la cellule indiquant l'atteinte de la capacité de saturation en eau de la charge de tamis intervient après 18h30 de fonctionnement, ce qui correspond à une capacité de saturation en eau de 19,9 % par rapport au poids de tamis sec .

### Exemple 2 :

### Régénération de la charge de tamis moléculaire de type 3 A par chauffage à 200°C , sous pression de 1 mm Hg :

L' ensemble décrit sur la figure 1 permet également de réaliser, pour une même charge de tamis moléculaire, plusieurs cycles ; chaque cycle comprenant le séchage en continu de F32 humide jusqu'à saturation en eau de la charge de tamis, suivi de la régénération de ladite charge. Ces cycles sont réalisés avec une consommation minimale de F32.

Après les 18h30 de fonctionnement et l'atteinte de la capacité de saturation en eau de la charge de tamis tels que décrits à l'exemple 1, on arrête la circulation du courant gazeux humide de F32, en fermant les vannes appropriées.

On fait ensuite circuler dans le tube sécheur (6) un courant d'hélium pendant 2 heures à température ambiante, cette opération ayant pour but l'élimination du F32 restant entre les granulés de la charge de tamis moléculaire.

On ferme alors les vannes (18) et (19), et on ouvre la vanne (22), de manière à relier le tube sécheur (6) à une pompe à vide, par l'intermédiaire d'un piège métallique plongé dans l'azote liquide.

La pression dans ledit tube est ainsi abaissée à une valeur de 1 mm Hg. La température dans le tube sécheur (6) est fixée à 200°C par circulation d'un fluide caloporteur dans la double enveloppe dudit sécheur.

Ces conditions de température et de pression sont maintenues pendant environ 2 heures, jusqu'à désorption complète de l'eau et de la petite quantité de F32 encore adsorbée dans la charge de tamis. L'eau (et le F32) ainsi désorbés sont retenus dans le piège à l'azote liquide, dont le poids est déterminé à intervalles de temps réguliers. Le traitement de régénération est arrêté lorsque le poids du piège est sensiblement constant.

L'essai de séchage tel que défini dans l'exemple 1 est alors répété avec la charge de tamis ainsi régénérée.

La capacité de saturation en eau de la charge de tamis est atteinte au bout de 19 heures de fonctionnement. Elle est de 18,4 %, et représente donc 92,5 % de la capacité de saturation en eau déterminée à la fin de l'exemple 1.

Cet exemple montre donc que la capacité de saturation en eau est maintenue à une valeur sensiblement constante après le traitement de régénération, avantageusement compris dans le procédé de séchage de F32 humide selon l'invention.

### Exemple 3 :

### Régénération d'une charge de tamis moléculaire de type 3 A par un courant d'hélium effectuée avec 3 paliers de température :

On répète l'exemple 1 avec une charge fraîche de 41,1 g de tamis moléculaire Ceca NK 30 (3 Å).

La capacité de saturation est atteinte après 19 heures de fonctionnement. Elle est de 18,5 % par rapport au poids de tamis sec.

On procède alors à la régénération de la charge de tamis en faisant circuler dans le tube sécheur (6) un courant d'hélium à la pression atmosphérique normale et dans les conditions suivantes :
- à 120°C, durant 2 heures, puis
- à 150°C durant 1 heure 30, puis
- à 200°C durant 2 heures.

Le suivi chromotographique (14) du F32 désorbé montre que les quantités de F32 correspondantes (exprimées par rapport à la quantité de F32 initiale adsorbée) sont d'environ 70 % à l'issue du palier à 120°C, d'environ 90 % à l'issue du palier à 150°C.

Le suivi chromotographique (14) de l'eau dans le courant d'He montre qu'à l'issue du palier à 200°C, on a désorbé plus de 95 % de l'eau adsorbée sur la charge de tamis.

L'essai de séchage tel que défini dans l'exemple 1 est alors répété avec la charge de tamis ainsi régénérée.

On mesure une capacité de saturation en eau de 16,9 %. Une telle valeur correspond à 91,3 % de la capacité de saturation en eau atteinte à la fin de l'étape de séchage du présent exemple.

Cet exemple montre donc que la capacité de saturation en eau est maintenue à une valeur sensiblement constante après le traitement de régénération, avantageusement compris dans le procédé de séchage de F32 humide selon l'invention.

### Exemple 4 :

On utilise 40,3 g d'une charge de tamis non fraîche, dont la capacité de saturation en eau, préalablement déterminée selon l'exemple 1, est de 14,2 %.

A partir de cette charge de tamis, on réalise une série de cycles séchage/régénération ; chaque cycle comprend le séchage en continu du F32 humide réalisé conformément à l'exemple 1 jusqu'à saturation en eau de la charge, et suivi de la régénération de ladite charge conformément au programme de températures suivant :
- 3 heures à 120 °
- 3 heures à 160 °
- 2 heures à 200 °

On constate que 98 % de la quantité de F32 initiale adsorbée est désorbée à l'issue des 3 heures à 160°C.

Les résultats sont rassemblés dans le tableau suivant. Ils montrent que la capacité de saturation en eau de la charge de tamis est maintenue sensiblement constante.

| Cycle n° | Capacité en eau (%) |
|---|---|
| 1 | 14,2 |
| 2 | 14,3 |
| 3 | 14,5 |
| 4 | 13,0 |
| 5 | 15,3 |

### Exemple comparatif :

On répète l'exemple 1 avec une charge fraîche de tamis de 40,8 g.

La capacité de saturation en eau est atteinte après 19h de fonctionnement. Elle est de 18,4 % par rapport au poids de tamis sec.

La régénération est réalisée sous balayage d'hélium séché (12) et préchauffé à 150°C (four 13), le lit de tamis étant chauffé simultanément par la double enveloppe du sécheur (6) ; ces modalités ont pour but d'atteindre très rapidement une température de 200°C sur l'ensemble de la charge de tamis comme cela se passe en pratique dans un procédé industriel.

Après un palier de 2 heures à cette température de 200°C, le tamis est refroidi et 35,3 g de cette charge sont soumis à l'essai de séchage tel que défini dans l'exemple 1.

La capacité de saturation en eau de la charge de tamis est atteinte dans ce cas au bout de 10 heures de fonctionnement et est de 11,2 % seulement. Cette valeur correspond à une baisse de 40 % par rapport à la capacité de saturation initiale, avant régénération.

## Revendications

1. Procédé de séchage de F32 humide comprenant la mise en contact en continu d'un courant dudit F32 avec une charge d'une composition comprenant un tamis moléculaire choisi parmi un tamis de type 3A, 4A ou 5A, à une température comprise entre 5 et 78°C, de préférence à température ambiante, et à une pression comprise entre 0,6 et 25 atm, de préférence entre 0,8 et 17 atm.

2. Procédé selon la revendication 1, **caractérisé en ce que** le courant de F32 à sécher est un courant gazeux, et la pression est comprise entre 0,6 et 10 atm, de préférence entre 0,8 et 5 atm.

3. Procédé selon l'une des revendications 1 ou 2, **caractérisé en ce que** le courant de F32 comprend une teneur en eau inférieure à 10000 ppm, de préférence inférieure à 6000 ppm.

4. Procédé selon l'une des revendications 1 à 3, **caractérisé en ce que** la mise en contact du F32 humide et de la charge de tamis est réalisée dans une colonne située en aval d'une unité de fabrication de F32.

5. Procédé selon l'une des revendications 1 à 4, **caractérisé en ce que** le tamis moléculaire mis en oeuvre est un tamis de type 3 A.

6. Procédé selon l'une des revendications 1 à 5, **caractérisé en ce que** la charge de tamis est régénérée par le procédé consistant à chauffer ladite charge à une température comprise entre 120°C et 300°C, de préférence entre 150° et 250°C, à une pression absolue inférieure à 100 mm Hg, de préférence inférieure à 80 mm Hg.

7. Procédé selon l'une des revendications 1 à 5, **caractérisé en ce que** la charge de tamis est régénérée par le procédé consistant à faire passer un courant d'un gaz inerte, tel que l'hélium, sur ladite charge, à une pression voisine de la pression atmosphérique, en opérant tout d'abord :
(i) à au moins une température comprise entre 70°C et 170°C, de préférence entre 80°C et 165 °C, durant le temps nécessaire à l'élimination d'au moins 80 %, de préférence au moins 90 %, de la quantité de F32 initiale adsorbée dans la charge, puis,
(ii) à une autre température comprise entre 180°C et 300°C, de préférence entre 190°C et 250°C, durant le temps nécessaire à l'élimination d'au moins 90 %, de préférence au moins 95 %, de la quantité d'eau initiale adsorbée dans la charge.

8. Procédé selon la revendication 7 **caractérisé en ce que** l'on met en oeuvre l'étape (i) en opérant tout d'abord :
- (i1) à une première température comprise entre 70°C et 130°C, de préférence entre 100°C et 125°C, durant le temps nécessaire à l'élimination d'au moins 60 % (de préférence au moins 70 %) de la quantité de F32 initiale adsorbée, puis
- (i2) à une deuxième température comprise entre 130°C et 170°C, de préférence entre 145°C et 165°C, durant le temps nécessaire à l'élimination d'au moins 80 %, de préférence au moins 90 %, de la quantité de F32 initiale adsorbée.

9. Procédé selon l'une des revendications 6 à 8, **caractérisé en ce que** le traitement de régénération de la charge de tamis est mis en oeuvre dans la même colonne que celle définie dans la revendication 4.

10. Procédé selon la revendication 9, **caractérisé en ce qu'**il est mis en oeuvre dans deux colonnes en parallèle, l'une fonctionnant en phase de séchage de F32 humide, l'autre fonctionnant en phase de régénération d'une charge de tamis moléculaire saturée.

## Claims

1. Process for drying wet F32, which comprises placing a stream of the said F32 in continuous contact with a charge of a composition comprising a molecular sieve chosen from a 3A, 4A or 5A type sieve, at a temperature of between 5 and 78°C, preferably at room temperature, and at a pressure of between 0.6 and 25 atm, preferably between 0.8 and 17 atm.

2. Process according to Claim 1, **characterized in that** the stream of F32 to be dried is a stream of gas, and the pressure is between 0.6 and 10 atm, preferably between 0.8 and 5 atm.

3. Process according to either of Claims 1 and 2, **characterized in that** the stream of F32 comprises a water content of less than 10,000 ppm, preferably less than 6000 ppm.

4. Process according to one of Claims 1 to 3, **characterized in that** the wet F32 is placed in contact with the sieve charge in a column located downstream of a plant for manufacturing F32.

5. Process according to one of Claims 1 to 4, **characterized in that** the molecular sieve used is a 3 A type sieve.

6. Process according to one of Claims 1 to 5, **characterized in that** the sieve charge is regenerated by the process which consists in heating the said charge to a temperature of between 120°C and 300°C, preferably between 150°C and 250°C, at an absolute pressure of less than 100 mmHg, preferably less than 80 mmHg.

7. Process according to one of Claims 1 to 5, **characterized in that** the sieve charge is regenerated by the process which consists in passing a stream of an inert gas, such as helium, over the said charge, at a pressure in the region of atmospheric pressure, by working firstly:
(i) at a temperature at least between 70°C and 170°C, preferably between 80°C and 165°C, for the time required to remove at least 80%, preferably at least 90%, of the initial amount of F32 adsorbed in the charge, and then
(ii) at another temperature of between 180°C and 300°C, preferably between 190°C and 250°C, for the time required to remove at least 90%, preferably at least 95%, of the initial amount of water adsorbed in the charge.

8. Process according to Claim 7, **characterized in that** step (i) is carried out by first working:
- (i1) at a first temperature of between 70°C and 130°C, preferably between 100°C and 125°C, for the time required to remove at least 60% (preferably at least 70%) of the initial amount of F32 adsorbed, and then
- (i2) at a second temperature of between 130°C and 170°C, preferably between 145°C and 165°C, for the time required to remove at least 80%, preferably at least 90%, of the initial amount of F32 adsorbed.

9. Process according to one of Claims 6 to 8, **characterized in that** the regeneration treatment for the sieve charge is carried out in the same column as that defined in Claim 4.

10. Process according to Claim 9, **characterized in that** it is carried out in two columns in parallel, one running in the phase for drying wet F32, the other running in the phase for regenerating a saturated molecular sieve charge.

## Patentansprüche

1. Verfahren zum Trocknen von feuchtem F32, das umfasst, einen Strom von F32 mit einer Charge einer Zusammensetzung, die ein Molekularsieb enthält, das unter den Sieben vom Typ 3A, 4A oder 5A ausgewählt ist, bei einer Temperatur im Bereich von 5 bis 78 °C und vorzugsweise bei Raumtemperatur und einem Druck von 0,6 bis 25 atm und vorzugsweise 0,8 bis 17 atm kontinuierlich in Kontakt zu bringen.

2. Verfahren nach Anspruch 1, **dadurch gekennzeichnet, dass** der zu trocknende F32-Strom ein Gasstrom ist und der Druck im Bereich von 0,6 bis 10 atm und vorzugsweise 0,8 bis 5 atm liegt.

3. Verfahren nach einem der Ansprüche 1 oder 2, **dadurch gekennzeichnet, dass** der F32-Strom einen Wassergehalt unter 10 000 ppm und vorzugsweise unter 6000 ppm aufweist.

4. Verfahren nach einem der Ansprüche 1 bis 3, **dadurch gekennzeichnet, dass** das feuchte F32 in einer Kolonne mit der Molekularsieb-Charge in Kontakt gebracht wird, die sich stromaufwärts einer Anlage zur Herstellung von F32 befindet.

5. Verfahren nach einem der Ansprüche 1 bis 4, **dadurch gekennzeichnet, dass** das verwendete Molekularsieb ein Sieb vom Typ 3A ist.

6. Verfahren nach einem der Ansprüche 1 bis 5, **dadurch gekennzeichnet, dass** die Molekularsieb-Charge nach einem Verfahren regeneriert wird, das darin besteht, die Charge auf eine Temperatur im Bereich von 120 bis 300 °C und vorzugsweise 150 bis 250 °C bei einem absoluten Druck unter 100 mm Hg und vorzugsweise unter 80 mm Hg zu erwärmen.

7. Verfahren nach einem der Ansprüche 1 bis 5, **dadurch gekennzeichnet, dass** die Molekularsieb-Charge nach einem Verfahren regeneriert wird, das darin besteht, einen Strom eines Inertgases wie Helium bei einem Druck in der Gegend von Atmosphärendruck durch die Charge zu leiten, wobei zunächst:
(i) bei einer Temperatur von mindestens 70 bis 170 °C und vorzugsweise 80 bis 165 °C während einer Zeitspanne, die zum Entfernen von mindestens 80 % und vorzugsweise mindestens 90 % der anfänglich an der Charge adsorbierten F32-Menge erforderlich ist, und anschließend
(ii) bei einer anderen Temperatur im Bereich von 180 bis 300 °C und vorzugsweise 190 bis 250 °C während einer Zeitspanne gearbeitet wird, die erforderlich ist, um mindestens 90 % und vorzugsweise mindestens 95 % der anfänglich an der Charge adsorbierten Wassermenge zu entfernen.

8. Verfahren nach Anspruch 7, **dadurch gekennzeichnet, dass** der Schritt (i) durchgeführt wird, indem zunächst:
- (i1) bei einer ersten Temperatur im Bereich von 70 bis 130 °C und vorzugsweise 100 bis 125 °C während einer Zeitspanne, die erforderlich ist, um mindestens 60 % (vorzugsweise mindestens 70 %) der anfänglich adsorbierten F32-Menge zu entfernen, und anschließend
- (i2) bei einer zweiten Temperatur im Bereich von 130 bis 170 °C und vorzugsweise 145 bis 165 °C während einer Zeitspanne gearbeitet wird, die erforderlich ist, um mindestens 80 % und vorzugsweise mindestens 90 % der anfänglich adsorbierten F32-Menge zu entfernen.

9. Verfahren nach einem der Ansprüche 6 bis 8, **dadurch gekennzeichnet, dass** die Regenerierung der Molekularsieb-Charge in der in Anspruch 4 definierten Kolonne durchgeführt wird.

10. Verfahren nach Anspruch 9, **dadurch gekennzeichnet, dass** es in zwei parallel geschalteten Kolonnen durchgeführt wird, wobei eine Kolonne in der Phase der Trocknung des feuchten F32 arbeitet und die andere Kolonne eine gesättigte Molekularsieb-Charge regeneriert.
